# EUROPÄISCHE PATENTSCHRIFT

(11) **EP 1 513 490 B1**
(45) Veröffentlichungstag und Bekanntmachung des Hinweises auf die Patenterteilung: **01.02.2012**
(21) Anmeldenummer: 03735498.2
(22) Anmeldetag: 30.05.2003
(51) Int. Cl.: A61Q 19/00, A61K 8/35, A61K 8/97, A61K 8/34, A61K 8/86

(54) **KOSMETISCHE ODER DERMATOLOGISCHE ZUBEREITUNGEN ENTHALTEND LICOCALCHON A ODER EINEN LICOCALCHON A ENTHALTENDEN EXTRAKT AUS RADIX GLYCYRRHIZAE INFLATAE**
COSMETIC OR DERMATOLOGICAL PREPARATIONS CONTAINING LICOCHALCONE A OR AN EXTRACT OF RADIX GLYCYRRHIZAE INFLATAE , CONTAINING LICOCHALCONE A
PREPARATIONS COSMETIQUES OU DERMATOLOGIQUES CONTENANT DE LA LICOCHALCONE A OU UN EXTRAIT CONTENANT DE LA LICOCHALCONE A PROVENANT DE RADIX GLYCYRRHIZAE INFLATAE

(30) Priorität: 01.06.2002 DE 10224387
(43) Veröffentlichungstag der Anmeldung: 16.03.2005
(73) Patentinhaber: Beiersdorf AG, 20245 Hamburg (DE)
(72) Erfinder: TOM DIECK, Karen, 22299 Hamburg (DE); KOLBE, Ludger, 21255 Dohren (DE); MUNDT, Claudia, 28207 Bremen (DE); WENSORRA, Ursula, 21035 Hamburg (DE); WOLBER, Rainer, 22397 Hamburg (DE)
(86) Internationale Anmeldenummer: PCT/EP2003/005660
(87) Internationale Veröffentlichungsnummer: WO 2003/101414

(56) Entgegenhaltungen:
- EP-A- 0 998 939
- PATENT ABSTRACTS OF JAPAN vol. 2000, no. 23, 10. Februar 2001 (2001-02-10) & JP 2001 163718 A (MARUZEN PHARMACEUT CO LTD), 19. Juni 2001 (2001-06-19)
- PATENT ABSTRACTS OF JAPAN vol. 017, no. 113 (C-1033), 9. März 1993 (1993-03-09) & JP 04 297418 A (SUMITOMO METAL IND LTD), 21. Oktober 1992 (1992-10-21)
- PATENT ABSTRACTS OF JAPAN vol. 2000, no. 11, 3. Januar 2001 (2001-01-03) & JP 2000 212060 A (NARIS COSMETICS CO LTD), 2. August 2000 (2000-08-02)
- KOBAYASHI M. ET AL.: "Antibacterial Activity of Licochalcone A against Spore-forming Bacteria" ANTIMICROBIAL AGENTS AND CHEMOTHERAPY, Bd. 46, Nr. 5, Mai 2002 (2002-05), Seiten 1226-1230, XP002254246
- PATENT ABSTRACTS OF JAPAN vol. 2002, no. 07, 3. Juli 2002 (2002-07-03) & JP 2002 080343 A (NIKKO SEIYAKU KK), 19. März 2002 (2002-03-19)
- Report 0426-2009
- KOLBE ET AL.: "Anti-inflammatory efficacy of Licochalcone A: correlation of clinical potency and in vitro effects", ARCH. DERMATOL. RES., 2006, DOI: 10.1007/s00403-006-0654-4
- Aus der Beiersdorf-Forschung, Neuer hautberuhigender Wirkstoff
- WEBER ET AL.: 'Skin tolerance, efficacy, and quality of life of patients with red facial skin using a skin care regimen containing Licochalcone A' JOURNAL OF COSMETIC DERMATOLOGY Bd. 5, Seiten 227 - 232
- Löslichkeiten von Polyol Soluble Licorice extract P-U von Maruzen / PK 7573
- Photoprotective properties of Licochalcone A on human skin in vitro and in vivo
- Ergebnisse Chalkone/Dihydrochalkone

## Beschreibung

Die vorliegende Erfindung betrifft Wirkstoffe zur Herstellung von kosmetischen oder dermatoldgischen Zubereitungen zur Prophylaxe und Behandlung von entzündlichen Hautzuständen und/oder zum Hautschutz bei empfindlich determinierter trockener Haut.

Ferner betrifft die Erfindung die Verwendung solcher Wirkstoffe und Zubereitungen, solche Wirkstoffe enthaltend, zur Immunstimulation der Haut, dabei vorteilhaft auch zur Immunstimulation im Sinne einer Behandlung der verletzten Haut, insbesondere zur Behandlung von Wunden.

Darüberhinaus betrifft die Erfindung Zubereitungen mit extrem niedrigem sogenanntem "Stinging Potential" sowie kosmetische oder dermatologische Formulierungen, welche die Haut - z. B. nach einem Sonnenbad - gezielt pflegen und die Nachreaktionen der Haut auf die Einwirkung von UV-Strahlung vermindern.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismus spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Immunsuppression im allgemeinen ist die Unterdrückung oder Abschwächung der Reaktivität des Immunsystems. Die Immunsuppression kann in lokale und systemische Effekte aufgegliedert werden. Letztlich umfaßt sie eine Vielzahl verschiedenster Aspekte, welche alle eine Reduktion der normalen immunologischen Abwehrmechanismen der Haut beinhalten. Es ist bekannt, das ultraviolettes (UV-) Licht, wie es im Sonnenlicht enthalten ist, zur Immunsupression führen kann. Bei Bestrahlung der Haut mit UV-Licht (insbesondere UVB-Licht) werden sowohl lokale wie auch systemische Aspekte der UVinduzierten Immunsupression beobachtet.

Bei Menschen mit sensibler, empfindlicher oder verletzlicher Haut kann ein mit "Stinging" (<engl.> "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

"Stinging"-Phänomene können als kosmetisch zu behandelnde Störungen angesehen werden. Starker Juckreiz dagegen, insbesondere bei Atopie auftretendes starkes Hautjucken sowie Juckreiz bei Hauterkrankungen, kann auch als schwerwiegendere dermatologische Störung bzw. neurosensorisches Phänomen bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen - z. B. Massage, Einwirkung (waschaktiver) Tenside, Wettereinfluß wie Sonne, Kälte, Trockenheit, aber auch feuchte Wärme, Wärmestrahlung und UV-Strahlung, z. B. der Sonne - hervorgerufen werden.

In "Journal of the Society of Cosmetic Chemists" 28, S.197 - 209 (Mai 1977) beschreiben P. J. Frosch und A. M. Kligman eine Methode zur Abschätzung des "Stinging-Potentials" topisch verabreichter Substanzen. Als positive Substanzen werden hier z. B. Milchsäure und Brenztraubensäure eingesetzt. Bei Messung nach dieser Methode wurden aber auch Aminosäure, insbesondere Glycin, als neurosensorisch aktiv ermittelt (solche Substanzen werden "Stinger" gebannt).

Nach bisherigen Erkenntnissen tritt eine derartige Empfindlichkeit gegenüber ganz bestimmten Substanzen individuell unterschiedlich auf. Dies bedeutet, daß eine Person, die bei Kontakt mit einer Substanz "Stingingeffekte" erhebt, sie mit hoher Wahrscheinlichkeit bei jedem weiteren Kontakt wiederholt erleben wird. Der Kontakt mit anderen "Stingern" kann aber ebensogut ohne jede Reaktion verlaufen.

Viele mehr oder weniger empfindliche Personen haben auch bei Verwendung mancher desodorierenden oder antitranspirierend wirkenden Zubereitungen unter erythematösen Hauterscheinungen zu leiden.

Erythematöse Hauterscheinungen treten ferner auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Auch die Rasur induziert in dafür empfindlichen Personen Erytheme, Brennen, Juckreiz und Spannungsgefühle, die durch die oberflächliche Verletzung und die mechanische Belastung der obersten Hautschichten sowohl bei der Nassrasur als auch bei der Trockenrasur ausgelöst werden. Diese Beschwerden treten häufig bei der täglichen Rasur der Barthaare auf, aber auch nach der Rasur von Achsel-, Scham-, und Beinbehaarung können Irritationen auftreten.

Neben den positiven Auswirkungen des Sonnenlichtes, wie dem allgemeinen Wohlbefinden, der Bildung von Vitamin D3 und der Aknebehandlung, gibt es auch negative Auswirkungen, denen es entgegenzuwirken gilt.

Die Bedingungen eines Sonnenbades stellen für den menschlichen Organismus eine ungewohnte, zum Teil extreme Belastung dar, von der insbesondere die Haut betroffen ist. Solange die Strahlenbelastung ein gewisses Ausmaß nicht überschreitet, wird unsere Haut damit fertig. Geringere Schäden, wie sie bei nicht spürbaren Suberythemen vorliegen, werden sofort behoben.
Setzt man die Haut allerdings zu lange der Sonne oder einer künstlichen Strahlenquelle aus, so entwickelt sich nach einer Latenzzeit von 2 bis 3 Stunden eine gegen die unbestrahlte Haut stark abgegrenzte Hautrötung, das Erythema solare. Bei dem so entstehenden Sonnenbrand unterscheidet man zwischen
■ 1. Grad: Erythem (Rötung, Wärmegefühl, Brennen, Spannungsgefühl der Haut) klingt nach 2 bis 3 Tagen wieder ab und verschwindet unter gleichzeitig zunehmender Pigmentierung,
■ 2. Grad: Blasenbildung
   auf der Haut bilden sich Blasen mit Brennen und Jucken, die Oberhaut wird flächig abgestoßen
■ 3. Grad: Zellschädigung
   es treten tiefgehende Zellschädigungen auf, der Körper reagiert mit Fieber, die Oberhaut wird großflächig abgestoßen.
Der 2. und 3. Grad werden auch als Dermatitis solare bezeichnet.

Die Bildung des Erythems ist abhängig von der Wellenlänge. Der Erythembereich des UV-B liegt zwischen 280 nm und 320 nm.

Etwa 90 % der auf die Erde gelangenden ultravioletten Strahlung besteht aus UV-A-Strahlen mit einer Wellenlänge zwischen 320 nm und 400 nm: Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Homschicht zurückgehalten werden.

Man hat lange Zeit fälschlicherweise angenommen, daß die langwellige UV-A-Strahlung nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlüng durch UV-A-Strahlung noch verstärkt werden.

Da die Beiträge der verschiedenen Wellenlängebereiche des UV-Lichtes zu lichtbedingten Hautveränderungen nicht vollständig geklärt sind, geht man heute verstärkt davon aus, daß vorbeugender Schutz sowohl gegen UV-A- als auch gegen UV-B-Strahlen, beispielsweise durch Auftrag von Lichtschutzfiltersubstanzen in Form einer kosmetischen oder dermatologischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der Sonnenstrahlung schützen.

Ein Sonnenbad wird von den meisten Menschen als angenehm empfunden, die nachtelligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel angewendet werden.

Der Sonnenbrand bzw. das Lichterythem sind die akuten Erscheinungsformen der Lichteinwirkung. Neben den bereits beschriebenen Wirkungen der UV-Strahlen kommt es in der Nachreaktion der Haut ferner zu einer verminderten Sebumproduktion und einem Austrocknen der Haut. Zur Behandlung der Haut dienen die sogenannten Aftersun-Präparate, deren Anwendung grundsätzlich nach jeder Sonnenexposition empfohlen wird. Dabei handelt es sich in der Regel um Emulsionen oder wäßrige Hydrogele, die neben üblichen Feuchthaltesubstanzen auch spezielle Wirkstoffe enthalten können, wie beispielsweise
■ entzündungslindernde und kühlende Stoffe,
■ lokal anaestesierende Stoffe und/oder
■ desinfizierende Stoffe, um mögliche Hautinfektionen zu verhindern.

Eingesetzt werden z. B. aus Pflanzen gewonnene entzündungslindernde bzw. -hemmende Wirkstoffe wie Azulen und Bisabolol (Kamille), Glycyrrhizin (Süßholzwurzel), Hamamelin (Hamamelis) oder Gesamtextrakte, z. B. aus Aloe vera oder Kamille. Diese zeigen bei leichteren Formen und lokal begrenzten Erythemreaktionen gewisse Erfolge. Gleiches gilt für Cremes mit einem hohen Gehalt an ätherischen Ölen oder Panthenol.

Aftersun-Präparate sind dazu bestimmt, die Haut nach dem Sonnenbad zu kühlen und ihr Feuchthaltevermögen zu verbessern, wobei die Vermittlung des Kühleffektes eine zentrale Rolle spielt. Dieser Kühleffekt wird beispielsweise durch hohe Mengen an Ethanol erzielt, welches beim Verteilen der Formulierung auf der Haut spontan verdunstet. Auch Hydrogele, O/W-Emulsionen (Lotionen) oder wäßrige Schüttelmixturen haben durch die Verdunstungskälte der wäßrigen Phase einen ausgeprägten Kühleffekt, der über eine lokale Gefäßverengung zu einer Entzündungsmilderung führt.

Aufgabe der vorliegenden Erfindung war es, den Nachteilen des Standes der Technik abzuhelfen und Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, insbesondere Dermatosen, aber auch des Erscheinungsbildes des "Stingings" zur Verfügung zu stellen.

Ferner sollten solche Wirkstoffe bzw. Zubereitungen, solche Wirkstoffe enthaltend, zur Verfügung gestellt werden, welche zur Immunstimulation der Haut, dabei vorteilhaft auch zur Immunstimulation im Sinne der die Wundheilung fördernden Wirkung, verwendet werden können.

Der Begriff. "Entzündung" ist ein relativ weitgefaßter und alter Begriff. Schon vor der Zeitenwende führte Aulus Celsus vier der fünf Kardinalzeichen der Entzündung ein: *rubor, tumor, calor und dolor* (Rötung, Schwellung, Hitze und Schmerz). Im 2. Jahrhundert definierte Galen von Pergamon das 5. Zeichen *functio laesa* (eingeschränkte Funktion). Insgesamt umfaßt die Entzündungsforschung 2000 Jahre, davon 200 Jahre auf zellulärem Level und 20 Jahre auf molekularem Level. Dabei wurde immer offensichtlicher das der Begriff uneinheitlich ist.

Entzündliche Erkrankungen sind gekennzeichnet durch Infiltrate von Entzündungszellen die jedoch sehr verschieden zusammengesetzt sein können. Psoriasis, eine entzündliche Hauterkankung, ist z. B. gekennzeichnet durch ein Infiltrat an oligoklonalen T-Zellen und polymorphkernigen. Granulozyten in scharf umgrenzten entzündlichen Plaques. Die involvierte Haut des atopischen Ekzems hingegen ist gekennzeichnet durch infiltrierende T-Zellen gegen Umweltantigene und Eosinophilen Granulozyten. So vielfältig wie die entzündlichen Erscheinungsformen sind auch die Therapien mit anti-entzündlichen Substanzen.

Keinesfalls kann man davon ausgehen, das eine Substanz die bei der einen entzündlichen Erkrankung eine sehr gute Wirkung zeigt auch bei anderen Entzündungen genauso wirken wird. Deshalb wird in diesem Bereich intensiv geforscht, viele dieser meist chronischen Erkrankungen sind bis heute nicht zufriedenstellend therapierbar. Am nächsten kommen einer solchen allumfassenden Wirkung die Kortikosteroide, aber wegen der zum Teil gravierenden Nebenwirkungen kommen sie für eine kontinuierliche und längere Anwendung nicht in Frage. Für kosmetische Anwendungen sind Kortikosteroide aus diesem Grunde sogar ganz verboten, hier mussen andere Substanzen eingesetzt werden.

Hautberuhigende Kosmetika werden bei akuten Hautirritationen eingesetzt, diese sind von den oben beschriebenen (chronischen) Entzündungen abzugrenzen. Ursache für Irritationen können z. B. physikalische Reize wie UV-Strahlung oder Rasur sein. Insbesondere im Frühstadium und bei geringer Reizhöhe gibt es kein Infiltrat aus Entzündungszellen (T-Zellen, Makrophagen, Granulozyten,...), sondern die betroffenen Hautzellen (hauptsächlich Keratinozyten und Fibroblasten) produzieren selber eine Fülle von pro-entzündlichen Mediatoren. Diese Mediatoren aktivieren die Zellen, induzieren Abwehr- und Reparaturmechanismen und locken in der Folge dann Entzündungszellen an. Ziel der hautberuhigenden Wirkung von Kosmetika muß es also sein die negativen Folgen der Irritation zu verhindern, ohne die notwendigen Reparaturmechanismen zu blockieren. Die bekannten anti-entzündlichen Substanzen, die vor allem auf die infiltrierenden Zellen wirken sollen sind deshalb nur bedingt als Modellsubstanzen brauchbar.

Es war überraschend und darin liegt die Lösung dieser Aufgaben, daß die Verwendung von Licochalcon A zur Herstellung dermatologischer Zubereitungen zur Pflege und/oder Linderung von Erythemen welche durch physikalische Reizung der Haut hervorgerufen wurden. den Nachteilen des Standes der Technik abhelfen würde.

Vorteilhaft ist insbesondere eine erfindungsgemäße Verwendung, dadurch gekennzeichnet, daß die Zubereitungen 0,0001 bis 5 Gew.-%, insbesondere 0,001 bis 1 Gew.-%, ganz besonders 0,005 bis 0,15 Gew.% Licochalcon A enthalten, bezogen auf das Gesamtgewicht der Zubereitung.

Vorteilhaft ist ferner insbesondere eine erfindungsgemäße Verwendung, dadurch gekennzeichnet, daß die Zubereitungen 0,001 bis 10 Gew.-%, insbesondere 0,05 bis 5 Gew.-%, ganz besonders 0,01 bis 2 Gew.-% an einem oder mehreren ethoxylierten oder propoxylierten Rohstoffen enthalten, bezogen auf das Gesamtgewicht der Zubereitung.

Vorteilhaft ist ferner insbesondere eine erfindungsgemäße Verwendung, dadurch gekennzeichnet, daß die Zubereitungen 0,001 bis 10 Gew.-%, insbesondere 0,05 bis 5 Gew.%, ganz besonders 0,01 bis 2 Gew.-% an einem oder mehreren Polyolen enthalten, bezogen auf das Gesamtgewicht der Zubereitung.

Vorteilhaft ist ferner insbesondere eine erfindungsgemäße Verwendung, dadurch gekennzeichnet, daß die Zubereitungen Licocatchon A als Bestandteil von pflanzlichen Extracten, insbesondere von *Radix Glycyrrhizae inflatae,* enthalten.

Die Zubereitungen gemäß der Erfindung sind in jeglicher Hinsicht überaus befriedigende Präparate, die sich durch eine hervorragende Wirkung auszeichnen. Bei Anwendung der erfindungsgemäß verwendeten Wirkstoffe bzw. kosmetischer oder topischer dermatologischer Zubereitungen mit einem wirksamen Gehalt an erfindungsgemäß verwendeten Wirkstoffen ist eine wirksame Behandlung, aber auch eine Prophylaxe von entzündlichen Hautzuständen - auch dem atopischen Ekzem - und/oder zum Hautschutz bei empfindlich determinierter trockener Haut möglich. Der erfindungsgemäße Wirkstoff bzw. kosmetische oder topische dermatologische Zubereitungen mit einem wirksamen Gehalt an erfindungsgemäßem Wirkstoff dient aber auch in überraschender Weise zur Beruhigung von empfindlicher oder gereizter Haut.

Es war für den Fachmann nicht vorauszusehen gewesen, daß die erfindungsgemäßen Zubereitungen
■ lichtstrapazierte Haut oder
■ von der Rasur strapazierte Haut besser pflegen,
■ die Nachreaktionen der Haut auf die Einwirkung von UV-Strahlung besser vermindern,
■ die vom Sonnenbaden gereizte Haut besser beruhigen,
■ leichten Sonnenbrand schneller zum Abklingen bringen würden,
■ besser als feuchtigkeitsspendende Zubereitungen wirken,
■ einfacher zu formulieren sein,
■ besser die Hautglättung fördern und
■ sich durch besser Pflegewirkung auszeichen würden
als die Zubereitungen des Standes der Technik.

Die Erfindung ist selbstverständlich nicht auf Zubereitungen beschränkt, welche nach dem Sonnenbad angewendet werden, sondern umfaßt naturgemäß alle kosmetischen und dermatologischen Anwendungen, bei welchen eine entzündungslindernde Wirkung gewünscht oder von Vorteil sein könnte.

Hier sei insbesondere auch der Rasurbrand genannt, wie er nach der Rasur häufig auftritt.

Gegenstand der Erfindung ist daher ferner die Verwendung kosmetischer oder dermatologischer Formulierungen mit einem Gehalt an
- einem wäßrigen Extrakt aus *Radix Glycyrrhizae inflatae*
- einem oder mehreren ethoxylierten oder propoxylierten Rohstoffen
- gegebenenfalls einem oder mehreren Polyolen,
im Rahmen der vorliegenden Offenbarung auch kollektiv "erfindungsgemäße Wirkstoffkombination" genannt - zur Pflege licht- und rasurstrapazierter Haut und/oder zur Linderung oder der Nachreaktionen der Haut auf die Einwirkung von UV-Strahlung und/oder der Rasur.

Die Pflanzenart *Glycyrrhiza inflata* gehört wie das in Europa offizinelle Süßholz *Glycyrrhl*za *glabra* der Gattung *Glycyrrhiza* an, die zur Pflanzenfamille der *Fabaceae* (Erbsengewächse) gehört. Die Droge *Radix Glycyrrhizae* inflatae, d.h., die Wurzel der Pflanze, ist, beispielsweise in der fernöstlichen Medizin, gebräuchlich. Die Verwendung der Droge als Entzündungshemmer ist ebenfalls bekannt.

Ein Bestandteil des wäßrigen Auszugs aus Radix Glycyrrhizae inflatae ist das Licochalcon A, welches sich durch die folgende Strukturformel auszeichnet:

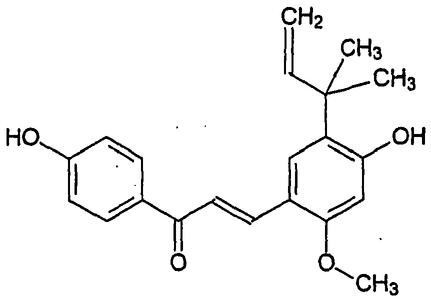

Es wird angenommen, daß diese Substanz, möglicherweise in Synergie mit den übrigen Bestandteilen des Extraktes, Anteil an der erfindungsgemäßen Wirkung besitzt.

Efindungsgemäß sind demnach auch kosmetische oder dermatologische Zubereitungen mit einem Gehalt an
- Licochalcon A
- Wasser
- einem oder mehreren ethoxylierten oder propoxylierten Rohstoffen
- sowie 0,001 bis 2 Gew.% Butylenglycol, bezogenaüf das Gesamtgewicht der Zubereitungen.

Es ist erfindungsgemäß vorteilhaft, wenn die kosmetischen oder dermatologischen Zubereitungen 0,001 bis 10 Gew.-%, insbesondere 0,05 bis 5 Gew.-%, ganz besonders 0,01 bis 2 Gew.-% an einem wäßrigen Extrakt aus *Radix Glycyrrhizae inflatae* enthalten, bezogen auf das Gesamtgewicht der Zubereitung.

Es ist erfindungsgemäß vorteilhaft, wenn die kosmetischen oder dermatologischen Zubereitungen 0,001 bis 10 Gew.-%, insbesondere 0,05 bis 5 Gew.-%, ganz besonders 0,01 bis 2 Gew.% an einem oder mehreren ethoxylierten oder propoxylierten Rohstoffen enthalten, bezogen auf das Gesamtgewicht der Zubereitung.

Insbesondere ist vorteilhaft, als ethoxylierten oder propoxylierten Rohstoffe das PPG-6 Decyltetradeceth-30 zu wählen.

Ganz besonders vorteilhaft ist es, von einem wäßrigen Extrakt auszugehen, der unter der Bezeichnung Aqua Licorlce Extract P-U der Firma Maruzen auszugehen, der ein wäßriges Gemisch (ca. 10 Gew.% Wasser) aus *Radix Glycyrrhizae Inflatae* (ca. 5 Gew.%, Anteil Licochalcone A im Extrakt ca. 22 %, PPG-6 Decyltetradeceth-30 (ca. 25 Gew.-%) und Butylenglycol (ca. 60 Gew.-%) darstellt.

Ferner ist es vorteilhaft, Licochalcone A in anderen Vehlkelsystemen in einer Konzentration von 0,0001 bis 5 Gew.-%, Insbesondere 0,001 bis 1 Gew.-%, ganz besonders 0,005 bis 0,15 Gew.-%, noch besonderer 0,005 - 0,05 Gew.-% zu verwenden.

Es ist bevorzugt Im Sinne der vorliegenden Erfindung, wenn die erfindungsgemäßen kosmetischen oder dermatologischen Zubereitungen einen oder mehrere Alkohole enthalten, insbesondere, wenn die Formulierungen in Form eines Aftersun-Präparats vorliegen und sich durch eine besondere Kühlwirkung auszeichnen sollen.

Die kosmetischen oder dermatologischen Formulierungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W-oder O/W/O-Emulsionen vorliegen. Solche Emulsionen können vorzugsweise auch eine Mikroemulsion, eine Pickering-Emulsion oder eine sprühbare Emulsion sein. Vorzugsweise enthalten die erfindungsgemäßen Formulierungen ferner weitere entzündungshemmende Substanzen, wie z. B. Allantoin, α-Bisabolol, Parithotensäure, Panthenol, Gelèe Royal, Kamillenextrakte, Azulen oder Aloe-vera-Extrakt sowie unverseifbare Anteile des Avocado- oder Sojaöls und weitere Substanzen, die die gereizte Haut beruhigen. Weitere vorteilhafte Wirkstoffe sind Gerbstoffe, welche adstringierende, entzündungshemmende und/oder sekretionshemmende Wirkung haben.

Darüberhinaus können die erfindungsgemäßen Formulierungen auch vorteilhaft Dihydroxyaceton oder Nußextrakte enthalten sowie weitere Substanzen, welche die Bräune erhalten sollen.

Die erfindungsgemäßen kosmetischen und/oder dermatologischen Formulierungen können wie üblich zusammengesetzt sein und insbesondere zur Behandlung und der Pflege der Haut und/oder der Haare nach einem Sonnenbad und als Schminkprodukt in der dekorativen Kosmetik dienen. Entsprechend können die erfindungsgemäßen Formulierungen - je nach ihrem Aufbau - beispielsweise verwendet werden als Hautschutzcrème; Reinigungsmilch, Sonnenschutzlotion, Nährcrème, Tages- oder Nachtcreme usw. Es ist gegebenenfalls möglich und vorteilhaft, die erfindungsgemäßen Formulierungen als Grundlage für pharmazeutische Formulierungen zu verwenden. Bevorzugt sind insbesondere solche kosmetischen und dermatologischen Formulierungen, die in der Form eines Aftersun-Hautpflegeproduktes oder eines After-shave-Produktes vorliegen.

Zur Anwendung werden die erfindungsgemäßen kosmetischen und dermatologischen Formulierungen in der für Kosmetika üblichen Weise, d. h. beispielsweise direkt - nach der Entnahme aus einer Flasche, Tube, einem Tiegel oder einem anderen Behältnis - oder mit Hilfe eines (getränkten) Tuches auf die Haut und/oder die Haare in ausreichender Menge aufgebracht.

Getränkte Tücher finden als Gegenstände des täglichen Bedarfs breiten Einsatz in unterschiedlichsten Bereichen. Sie erlauben unter anderem effiziente und hautschonende Reinigung und Pflege besonders auch in der Abwesenheit von (fließendem) Wasser. Dabei besteht der eigentliche Gebrauchsgegenstand aus zwei Komponenten:
a) einem trockenen Tuch, welches aus Materialien wie Papier und/oder unterschiedlichsten Mischungen aus Natur- oder Kunstfasern aufgebaut ist und
b) einer niederviskosen Tränküngslösung.

Gegenstand der vorliegenden Erfindung sind daher auch
kosmetische und dermatologische Tücher, welche mit kosmetischen oder dermatologischen Tränkungslösungen befeuchtet sind, die einen Gehalt an erfindungsgemäßer Wirkstoffkombination aufweisen.

Erfindungsgemäß bevorzugte "trockene" Tücher (gemäß a)) bestehen aus Vlies, insbesondere aus wasserstrahlverfestigten und/oder wasserstrahlgeprägten Vlies.

Derartige Vliese können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Es hat sich als vorteilhaft herausgestellt für das Tuch, wenn dieses ein Gewicht von 35 bis 120 g/m², vorzugsweise von 40 bis 60 g/m², hat (gemessen bei 20°C ± 2 °C und bei einer Feuchtigkeit der Raumluft von 65 % ± 5 % für 24 Stunden).

Die Dicke des Vlieses beträgt vorzugsweise 0,4 mm bis 2 mm, insbesondere 0,6 mm bis 0,9 mm.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sonden es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer besonders vorteilhaften Ausführungsform des Vlieses bestehen die Fasern aus einer Mischung aus 70 % Viskose und 30 % PET.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Fernen weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Es ist vorteilhaft im Sinne der vorliegenden Erfindung, wenn das Gewichtsverhältnis des ungetränkten Tuchs zu der Tränkungslösung aus dem Bereich von 2 : 1 bis 1 : 6 gewählt wird.

Die im Rahmen der Beschreibung der vorliegenden Erfindung genannten erfindungsgemäßen kosmetischen und dermatologischen Formulierungen bzw. Zubereitungen stellen vorteilhafte Tränklösungen für kosmetische und dermatologische Tücher im Sinne der vorliegenden Erfindung dar.

Es ist vorteilhaft, wenn die erfindungsgemäßen Tränklösungen dünnflüssig, insbesondere sprühbar sind und z. B. eine Viskosität von weniger als 2000 mPa•s, insbesondere weniger als 1.500 mPa•s haben (Meßgerät: Haake Viskotester VT-02 bei 25 °C).

Die erfindungsgemäßen kosmetischen und dermatologischen Formulierungen können kosmetische Hiifsstoffe enthatten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel und/oder Silikonderivate sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Homschicht (auch transepidermal water loss (TEWL) genannt) zu reduzieren und/oder die Hydratation der Homschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.
Glycerin kann als Moisturizer im Sinne der vorliegenden Anmeldung im Bereich von 0,05-30 Gew.%, besonders bevorzugt sind 1-10%, eingesetzt werden.

Die jeweils einzusetzenden Mengen an kosmetischen oder dermatologischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Camosin, D-Camosin, L-Camosin und deren Derivate (z. B. Anserin), Carotinoide, Carotine (z. B. α-Carotin, β-carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Melanine, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Alanindiessigsäure, Flavonoide, Polyphenole, Catechine, Vitamin C und Derivate (z. B. Ascorbylpalmitat, Mg-Ascorbylphosphat, Ascorbylacetat), Tocopherole und Derivate (z. B. Vitamin-E-acetat), sowie Koniferylbenzoat des Benzoäharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20 Gew.-%, insbesondere 0,1 bis 10 Gew.%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.%, bezogen auf das Gesamtgewicht der Formulierung, zu wählen.

Die Lipidphase kann vorteilhaft gewählt werden aus folgender Substanzgruppe:
- Mineralöls, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen:

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Formulierungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Xanthangummi und/oder Hydroxypropylmethylcellulose, jeweils einzeln oder in Kombination.

Es ist auch vorteilhaft im Sinne der vorliegenden Erfindung, kosmetische und dermatologische Zubereitungen zu erstellen, deren hauptsächlicher Zweck zwar nicht der Schutz vor Sonnenlicht ist, die aber dennoch einen Gehalt an UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und - gewünschtenfalls - Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise neben dem erfindungsgemäßen Wirkstoff zusätzlich mindestens eine weitere UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden.

Solche Pigmente können im Sinne der vorliegenden Erfindung vorteilhaft oberflächlich behandelt ("gecoatet") sein, wobei beispielsweise ein amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Erfindungsgemäß vorteilhaft sind z. B. Titandioxidpigmente, die mit Octylsilanol beschichtet sind. Geeignete Titandioxidpartikel sind unter der Handelsbezeichnung T805 bei der Firma Degussa erhältlich. Besonders vorteilhaft sind ferner mit Aluminiumstearat beschichtete TiO₂-Pigmente, z. B. die unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCl: Bisoctyltriazol], welches unter der Handelsbezeichnung Tinosorb® M bei der CIBA-Chemikallen GmbH erhältlich ist.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegende Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCl-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bomylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-sulfonsäure) bezeichnet wird.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate. Insbesondere bevorzugt sind das 2,4-Bis-{[4-(2-Ethyl-hexyloxy)-2-hydroxy]-phenyl}-6-(4-methoxyphenyl)-1,3,5-triazin (INCl: Aniso Triazin), welches unter der Handelsbezeichnung Tinosörb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 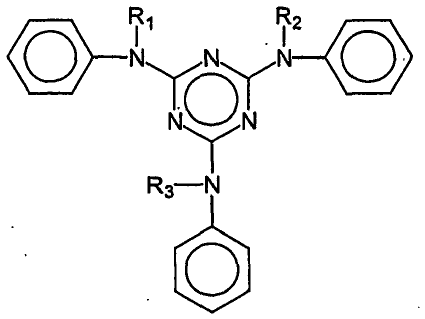 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 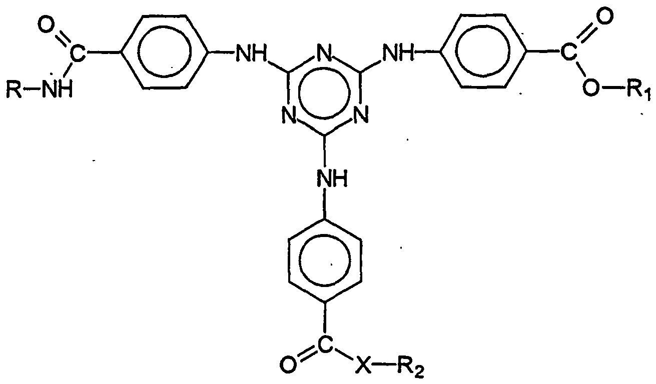 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten -C₁-C₁₈-Alkylrest, einen C₅-C₁₂-cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 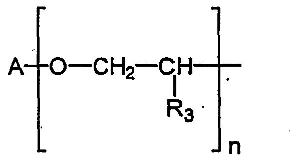 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cyclaalkylrest, gegebenenfalls substituiert mit einer oder mehreren C₁-C₄- Alkylgruppen, darstellt, wenn X die NH-Gruppe darstellt, und

einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 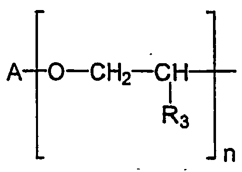 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen, .
- R₃: ein Wasserstoffatom oder eine Methylgruppe darstellt,
- n: eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 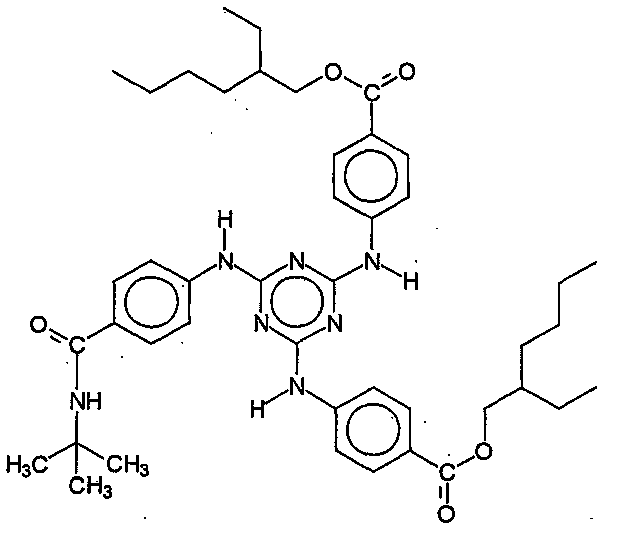 wiedergegeben wird, welches im Folgenden auch als Dioctylbutylamidotriazon (INCl: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoësäure-tris(2-etlhylhexylester), synonym: 2,4,6-Tris-[anilino-(p-carbo-2'-etlhyl-1'-hexyloxy)]-1,3,5-triazin (INCl: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 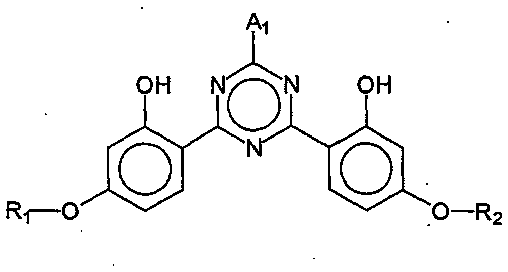 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1 ,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCl-Bezeichnung Drometrizole Trisiloxane.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester,
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des .Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Eine weiterere erfindungsgemäß vorteilhaft zu verwendende Lichtschutzhltersubstanz ist das Ethylhexyl-2-cyano-3,3-diphenylacrylat (Octocrylen), welches von BASF unter der Bezeichnung Uvinul^{®} N 539 erhältlich ist.

Es kann auch von erheblichem Vorteil sein, polymergebundene oder polyrnere UV-Filter substanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A-und/oder UV-B-Filter in kosmetische oder dermatologische Zubereitungen einzuarbeiten, beispielsweise bestimmte Salicylsäurederivate wie 4-lsopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiele O/W-Crèmes**

| Beispiel Nr. | 1 | 2 | 3 | 4 | 5 | 6 |
|---|---|---|---|---|---|---|
| Glycerylstearatcitrat | | | 2,00 | 2,00 | | 2,00 |
| Glycerylsterat selbstemulgierend | 4,00 | 3,00 | | | - | |
| PEG-40-Stearat | 1,00 | | | | | |
| Polyglyceryl-3-Methylglucose-Distearat | | | | | 3,00 | |
| Sorbitanstearat | | | | | | 2,00 |
| Stearinsäure | | 1,00 | | | | |
| Stearylalkohol | | | 2,00 | 2,00 | | |
| Cetylalkohol | 3,00 | 2,00 | | | 3,00 | |
| Cetylstearylalkohol | | | | | | 2,00 |
| Lanolinalkohol | | | 1,00 | 1,00 | | |
| Caprylic-/Capric-Triglycerid | 5, 00 | 3,00 | 4,00 | 4,00 | 3,00 | 3,00 |
| Octyldodecariol | | | | | | 2,00 |
| Dicaprylylether | | 4,00 | | | 2,00 | 1,00 |
| Paraffinum liquidum | 5,00 | 2,00 | 8,00 | 8,00 | 3,00 | |
| Dimethicon | | | 1,00 | 1,00 | | |
| Aqua Licorice Extract P-U | 0,25 | 0,05 | 0,15 | 0,15 | 1,00 | 0,05 |
| Tocopherol | 0,1 | | | | | 0,20 |
| Na₃HEDTA | 0,1 | | | | 0,1 | |
| Konservierungsmittel | q.s. | q.s. | | | q.s. | q.s. |
| Polyacrylsäure | 3,00 | 0,1 | | | 0,1 | 0,1 |
| Natronlauge 45% | q.s | q.s. | q.s. | q.s. | q.s. | q.s. |
| Glycerin | 5,00 | 3,00 | 7,50 | 7,50 | 3,00 | 3,00 |
| Butylenglycol | | 3,00 | | | | |
| Dihydroxyaceton | | | | 1,00 | | |
| Parfum | q.s. | q.s.. | q.s. | q.s. | q.s. | q.s. |
| Wasser | ad100 | ad100 | ad100 | ad100 | ad100 | ad100 |

**Beispiele O/W-Crèmes**

| Beispiel Nr. | 7 | 8 | 9 | 10 | 11 | |
|---|---|---|---|---|---|---|
| Glycerylstearatcitrat | | 2,00 | 2,00 | | | |
| Glycerylsterat selbstemulgierend | 5,00 | | | | | |
| Stearinsäure | | | | 2,50 | 3,50 | |
| Stearylalkohol | 2,00 | | | | | |
| Cetylalkohol | | | | 3,00 | 4,50 | |
| Cetylstearylalkohol | | 3,00 | 1,00 | | 0,50 | |
| C₁₂₋₁₅ Alkylbenzoat | | 2,00 | 3,00 | | | |
| Caprylic-/Capric-Triglycerid | 2,00 | | | | | |
| Octyldodecanol | 2,00 | 2,00 | | 4,00 | 6,00 | |
| Paraffinum liquidum | | 4,00 | 2,00 | | | |
| Cyclisches Dimethylpolysiloxan | | | | 0,50 | 2,00 | |
| Dimethicon Polydimethylsiloxan | 2,00 | | | | | |
| Titandioxid | 2,00 | | | | | |
| 4-Methylbenzyliden Campher | 1,00 | | | | 1,00 | |
| Butylmethoxy-dibenzoylmethan | 0,50 | | | | 0,50 | |
| Aqua Licorice Extract P-U | 0,08 | 0,50 | 0,25 | 1,00 | 0,40 | |
| 2,4-Bis-(4-(2-ethyl-hexyloxy-)2-hydroxyl)-phenyl)-6-(4-methoxyphenyl)-(1,3,5)-triazin | | 1,0 | 3,0 | | 0,5 | |
| Dihydroxyaceton | | 0,5 | | | 0,5 | |
| Tocopherol | | | | | 0,05 | |
| Ethylendiamintetraessigsäure Trinatrium | | | 0,20 | | 0,20 | |
| Konservierungsmittel, Parfum | q.s. | q.s. | q.s. | q.s. | q.s. | |
| Lanthan Gummi | | | 0,20 | | | |
| Polyacrylsäure | 0,15 | 0,1 | | 0,05 | 0,05 | |
| Natronlauge 45% | q.s. | q.s. | q.s. | q.s. | q.s. | |
| Glycerin | 3,00 | | 3,00 | 5,00 | 3,00 | |
| Butylenglycol | | 3,00 | | | | |
| Ethanol | | 3,00 | | 3,00 | | |
| Wasser | ad 100 | ad 100 | ad 100 | ad 100 | ad 100 | |

**Beispiele W/O-Emulsionen**

| Beispiel Nr. | 12 | 13 | 14 | 15 | 16 |
|---|---|---|---|---|---|
| Cetyldimethiconcopolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| 2-Ethylhexyl Methoxyzinnamat | | 8,00 | | 5,00 | 4,00 |
| 2,4-Bis-(4-(2-ethy)-hexyloxy-)2-hydroxyl)-phenyl)-6-(4-methoxyphenyl)-(1,3,5)-triazin | 2,00 | 2,50 | | 2,00 | 2,50 |
| Butylmethoxy-dibenzoylmethan | | | 2,00 | 1,00 | |
| Diethylhexyl Butamidotriazon | 3,00 | 1,00 | | | 3,00 |
| Ethylhexyl Triazon | | | 3,00 | 4,00 | |
| 4-Methylbenzyliden Campher | | 2,00 | | 4,00 | 2,00 |
| Octocrylen | 7,00 | 2,50 | 4,00 | | 2,50 |
| Diethylhexyl Butamidotriazon | 1,00 | | | 2,00 | |
| Phenylen-1,4-bis-(mononatrium,-2-benzimi dazyl-5,7-disulfonsäüre) | 1,00 | 2,00 | 0,50 | | |
| Phenylbenzimidazol Sulfonsäure | 0,50 | | | 3,00 | 2,00 |
| Titandioxid | | 2,00 | 1,50 | | 3,00 |
| Zinkoxid | 3,00 | 1,00 | 2,00 | 0,50 | |
| Paraffinum liquidum | | | 10,0 | | 8,00 |
| C₁₂₋₁₅ Alkyl-Benzoat | | | | 9,00 | |
| Dicaprylylether | 10,00 | | | | 7,00 |
| Butylen-Glycol-Dicaprylat/-Dicaprat | | | 2,00 | 8,00 | 4,00 |
| Dicaprylylcarbonat | 5,00 | | 6,00 | | |
| Dimethicon Polydimethylsiloxan | | 4,00 | 1,00 | 5,00 | |
| Phenylmethylpolysiloxan | 2,00 | 25,00 | | | 2,00 |
| Shea Butter | | | 3,00 | | |
| PVP Hexadecencopolymer | 0, 50 | | | 0,50 | 1,00 |
| Octoxyglycerin | | 0,30 | 1,00 | | 0,50 |
| Glycerin | 3,00 | 7,50 | | 7,50 | 2,50 |
| Glycinsoja | | 1,00 | 1,50 | | |
| Magnesiumsulfat | 1,00 | 0,50 | | 0,50 | |
| Magnesiumchlorid | | | 1,00 | | 0,70 |
| Tocopherolacetat | 0,50 | | 0,25 | | 1,00 |
| Aqua Licorice Extract P-U | 0,15 | 0,08 | 0,5 | 1,00 | 0,80 |
| Konservierungsmittel, Parfum | q.s. | q.s. | q.s. | q.s. | q.s. |
| Ethanol | 3,00 | | 1,50 | | 1,00 |
| Wasser | ad 100 | ad 100 | ad 100 | ad 100 | ad 100 |

**Beispiele W/O-Emulsionen**

| Beispiel Nr. | 17 | 18 |
|---|---|---|
| Polyglyceryl-2-dipolyhydroxystearat | 4,00 | 5,00 |
| Lanolinalkohol | 0,50 | 1,50 |
| Isohexadecan | 1,00 | 2,00 |
| Myristyl-Myristat | 0, 50 | 1,50 |
| Vaseline | 1,00 | 2,00 |
| Butylmethoxy-dibenzoylmethan | 0,50 | 1,50 |
| 4-Methylbenzyliden Campher | 1,00 | 3,00 |
| Butylen-Glycol-Dicaprylat/-Dicaprat | 4,00 | 5,00 |
| Shea Butter | - | 0,50 |
| Butylenglycol | - | 6,00 |
| Octoxyglycerin | - | 3,00 |
| Glycerin | 5,00 | - |
| Tocopherolacetat | 0,50 | 1,00 |
| Aqua Licorice Extract P-U | 0,2 | 0,1 |
| EDTA | 0,20 | 0,20 |
| Konservierungsmittel | q.s. | q.s. |
| Ethanol | - | 3,00 |
| Parfum | q.s. | q.s. |
| Wasser | ad 100 | ad 100 |

**Beispiel (W/O-Creme)**

| Beispiel Nr. | 19 |
|---|---|
| Polyglyceryl-3-Diisostearat | 3,50 |
| Glycerin | 3,00 |
| Polyglyceryl-2-Dipolyhydroxystearat | 3,50 |
| Aqua Licorice Extract P-U | 0,25 |
| Konservierungsmittel | q.s. |
| Parfum | q.s. |
| Magnesiumsulfat | 0,6 |
| Isopropylstearat | 2,0 |
| Caprylylether | 8,0 |
| Cetearylisononanoat | 6,0 |
| Wasser | ad 100 |

**Beispiel (W/O-Emulsion):**

| Beispiel Nr. | 20 |
|---|---|
| Triceteareth-4-Phosphat | 0,80 |
| Butylhydroxytoluol | 0,05 |
| Glyceryllanolat | 1,70 |
| Cyclomethicon | 2,20 |
| Isopropylpalmitat | 1,00 |
| Aqua Licorice Extract P-U | 0,50 |
| Polyacrylsäure | 0,50 |
| Ethylendiamintetraessigsäure | 1,00 |
| Natriumhydroxid | q.s. |
| Zitronensäure | 0,01 |
| Konservierungsmittel | q.s. |
| Parfum | q.s. |
| Wasser | ad 100 |

## Patentansprüche

1. Verwendung von Licochalcon A zür Hergstellung dermatologischer Zubereitungen zur Pflege und/oder Linderung von Erythemen, welche durch physikalische Reizung der Haut hervorgerufen wurden.

2. Verwendung nach Anspruch 1, **dadurch gekennzeichnet, daß** die Zubereitungen 0,0001 bis 5 Gew.-%, insbesondere 0,001 bis 1 Gew.-%, ganz besonders 0,005 bis 0,15 Gew.% Licochalcon A enthalten, bezogen auf das Gesamtgewicht der Zubereitung.

3. Verwendung nach Anspruch 1 oder 2, **dadurch gekennzeichnet, daß** die Zubereitungen 0,001 bis 10 Gew.-%, insbesondere 0,05 bis 5 Gew.%, ganz besonders 0,01 bis 2 Gew.-% an einem oder mehreren ethoxylierten oder propoxylierten Rohstoffen enthalten, bezogen auf das Gesamtgewicht der Zubereitu ng.

4. Verwendung nach einem oder mehreren der vorstehenden Ansprüche, **dadurch gekennzeichnet, daß** die Zubereitungen 0,001 bis 10 Gew.%, insbesondere 0,05 bis 5 Gew.-%, ganz besonders 0,01 bis 2 Gew.% an einem oder mehreren Polyolen enthalten, bezogen auf das Gesamtgewicht der Zubereitung.

5. Verwendung nach einem oder mehreren der vorstehenden Ansprüche, **dadurch gekennzeichnet, daß** die Zubereitungen Licocalchon A als Bestandteil von pflanzlichen Extrakten, insbesondere von *Radix Glycyrrhizae inflatae,* enthalten.

6. Verwendung nach einem oder mehreren der vorstehenden Ansprüche, **dadurch gekennzeichnet, daß** die Zubereitungen 0,0001 bis 5 Gew.-%, insbesondere 0,001 bis 1 Gew.-%, ganz besonders 0,005 bis 0,15 Gew.-% an einem wäßrigen Extrakt aus *Radix Glycyrrhizae inflatae* enthalten, bezogen auf das Gesamtgewicht der Zubereitung.

7. Verwendung nach einem oder mehreren der vorstehenden Ansprüche, **dadurch gekennzeichnet, daß** die Erytheme durch Lichteinwirkung, insbesondere UV-Licht, oder durch eine Rasur bedingt sind.

8. Kosmetische Zubereitungen, enthaltend Licochalcon A wasser einen oder mehrere ethoxylierte oder propoxylierte Rohstoffe sowie 0,001 bis 2 Gew.% an Butylenglycol bezogen auf das Gesamtgewicht der Zubereitung.

9. Zubereitungen nach Anspruch 8, enthaltend 0,0001 bis 5 Gew.-%, insbesondere 0,001 bis 1 Gew.-%. ganz besonders 0,005 bis 0,15 Gew.-% Licochalcon A, bezogen auf das Gesamtgewicht der Zubereitung.

10. Zubereitungen nach Anspruch 8 oder 9, **dadurch gekennzeichnet, daß** sie Licocalchon A als Bestandteil von pflanzlichen Extrakten, insbesondere von *Radix Glycyrrhizae inflatae,* enthalten.

## Claims

1. Use of licochalcone A for producing dermatological preparations for the care and/or alleviation of erythemas which have been caused by physical irritation of the skin.

2. Use according to Claim 1, **characterized in that** the preparations comprise 0.0001 to 5% by weight, in particular 0.001 to 1% by weight, very particularly 0.005 to 0.15% by weight, of licochalcone A, based on the total weight of the preparation.

3. Use according to Claim 1 or 2, **characterized in that** the preparations comprise 0.001 to 10% by weight, in particular 0.05 to 5% by weight, very particularly 0.01 to 2% by weight, of one or more ethoxylated or propoxylated raw materials, based on the total weight of the preparation.

4. Use according to one or more of the preceding claims, **characterized in that** the preparations comprise 0.001 to 10% by weight, in particular 0.05 to 5% by weight, very particularly 0.01 to 2% by weight, of one or more polyols, based on the total weight of the preparation.

5. Use according to one or more of the preceding claims, **characterized in that** the preparations comprise licochalcone A as constituent of plant extracts, in particular of *Radix Glycyrrhizae* inflatae.

6. Use according to one or more of the preceding claims, **characterized in that** the preparations comprise 0.0001 to 5% by weight, in particular 0.001 to 1% by weight, very particularly 0.005 to 0.15% by weight, of an aqueous extract from Radix *Glycyrrhizae inflate,* based on the total weight of the preparation.

7. Use according to one or more of the preceding claims, **characterized in that** the erythemas are caused by the action of light, in particular UV light, or by shaving.

8. Cosmetic preparations comprising licochalcone A, water, one or more ethoxylated or propoxylated raw materials, and 0.001 to 2% by weight of butylene glycol, based on the total weight of the preparation.

9. Preparations according to Claim 8, comprising 0.0001 to 5% by weight, in particular 0.001 to 1% by weight, very particularly 0.005 to 0.15% by weight, of licochalcone A, based on the total weight of the preparation.

10. Preparations according to Claim 8 or 9, **characterized in that** they comprise licochalcone A as constituent of plant extracts, in particular of *Radix Glycyrrhizae inflatae*.

## Revendications

1. Utilisation de Licochalcone A pour la préparation de compositions dermatologiques pour le soin et/ou le soulagement d'érythèmes qui ont été provoqués par irritation physique de la peau.

2. Utilisation selon la revendication 1, **caractérisée en ce que** les compositions contiennent 0,0001 à 5% en poids, en particulier 0,001 à 1% en poids, tout particulièrement 0,005 à 0,15% en poids de Licochalcone A, par rapport au poids total de la composition.

3. Utilisation selon la revendication 1 ou 2, **caractérisée en ce que** les compositions contiennent 0,001 à 10% en poids, en particulier 0,05 à 5% en poids, tout particulièrement 0,01 à 2% en poids d'une ou de plusieurs matières premières éthoxylées ou propoxylées, par rapport au poids total de la composition.

4. Utilisation selon l'une ou plusieurs des revendications précédentes, **caractérisée en ce que** les compositions contiennent 0,001 à 10% en poids, en particulier 0,05 à 5% en poids, tout particulièrement 0,01 à 2% en poids d'un ou de plusieurs polyols, par rapport au poids total de la composition.

5. Utilisation selon l'une ou plusieurs des revendications précédentes, **caractérisée en ce que** les compositions contiennent de la Licochalcone A comme constituant d'extraits végétaux, en particulier de Radix Glycyrrhizae inflatae.

6. Utilisation selon l'une ou plusieurs des revendications précédentes, **caractérisée en ce que** les compositions contiennent 0,0001 à 5% en poids, en particulier 0,001 à 1% en poids, tout particulièrement 0,005 à 0,15% en poids d'un extrait aqueux de Radix Glycyrrhizae inflatae, par rapport au poids total de la composition.

7. Utilisation selon l'une ou plusieurs des revendications précédentes, **caractérisée en ce que** les érythèmes sont dus à l'influence de la lumière, en particulier la lumière UV, ou à un rasage.

8. Compositions cosmétiques, contenant de la Licochalcone A, de l'eau, une ou plusieurs matières premières éthoxylées ou propoxylées ainsi que 0,001 à 2% en poids de butylèneglycol, par rapport au poids total de la composition.

9. Compositions selon la revendication 8, contenant 0,0001 à 5% en poids, en particulier 0,001 à 1% en poids, tout particulièrement 0,005 à 0,15% en poids de Licochalcone A, par rapport au poids total de la composition.

10. Compositions selon la revendication 8 ou 9, **caractérisées en ce qu'**elles contiennent de la Licochalcone A comme constituant d'extraits végétaux, en particulier de Radix Glycyrrhizae inflatae.
